# EUROPEAN PATENT APPLICATION

(11) **EP 4 289 859 A1**
(43) Date of publication of application: **13.12.2023**
(21) Application number: 22178402.8
(22) Date of filing: 10.06.2022
(51) Int. Cl.: C07K 14/55, C07K 14/74, C07K 14/725, C12N 5/0783, A61K 35/17, A61K 38/20, A61P 37/06, C07K 19/00

(54) **FUSION PROTEIN FOR MAINTENANCE OF REGULATORY T-CELLS**

(71) Applicant: Medizinische Hochschule Hannover, 30625 Hannover (DE)
(72) Inventor: Jaeckel, Dr., Elmar, 30625 Hannover (DE); Noyan, Dr., Fatih, 30625 Hannover (DE)
(74) Representative: Taruttis, Stefan Georg

(57) **Abstract**

The present invention relates to a fusion protein that provides for maintenance of regulatory T-cells that are polyclonal, e.g. natural isolated antigen-specific Treg cells, and/or Treg cells generated by introduction of a nucleic acid construct for expression of FOXP3, and/or Treg cells which express a chimeric antigen receptor (CAR), which Treg cells in contact with the cognate antigen are activated for suppressive activity, as well as to Treg cells that express the fusion protein, wherein the Treg cells are polyclonal or the Treg cells express a CAR. The fusion protein comprises or consists of an optional secretory leader peptide, IL-2, preferably a linker peptide, and a membrane-spanning anchor, which fusion protein is also termed membrane-bound IL-2.

## Description

The present invention relates to a fusion protein that provides for maintenance of regulatory T-cells (Treg cells) that are polyclonal, e.g. natural isolated antigen-specific Treg cells, and/or Treg cells generated by introduction of a nucleic acid construct for expression of FOXP3, and/or Treg cells which express a chimeric antigen receptor (CAR), which Treg cells in contact with the cognate antigen are activated for suppressive activity, as well as to Treg cells that express the fusion protein, wherein the Treg cells are polyclonal or the Treg cells express a CAR. The fusion protein of the invention in polyclonal Treg cells or in Treg cells also expressing a CAR, makes the Treg cells suitable for use in the treatment of transplant rejections, e.g. for use in the treatment of HvG or GvH disease, and for use in the treatment of autoimmune diseases. In the embodiment of Treg cells expressing both the fusion protein and the CAR and/or FOXP3 from a nucleic acid construct, the fusion protein and one or both of the CAR and FOXP3 can be expressed as a joint fusion protein having a connecting protease site between the fusion protein and each of the optional CAR and the optional FOXP3.

The fusion protein has the advantage of maintaining viability and suppressive activity of Treg cells in an environment of very low concentrations of environmental IL-2 or absent environmental IL-2, which can especially be caused by or in combination with general immune suppression, e.g. in combination with an immune suppressant for use in immune suppression, especially for use in the treatment of transplant rejections or of autoimmune diseases. Therein, an immune suppressant can be a calcineurin inhibitor, e.g. Tacrolimus and/or cyclosporin A.

### State of the art

Amado et al., "IL-2 coordinates IL-2 producing and regulatory T cell interplay" J Exp Med (2013) 210(12): 2707-2720 describes that Treg cells, which do not produce IL-2, are strictly dependent on exogenous IL-2 for survival, whereas IL-2 is mainly produced and secreted by Teffector cells.

Sakaguchi et al. "Immunologic self-tolerance maintained by activated T cells expressing IL-2 receptor alpha-chains (CD25). Breakdown of a single mechanism of self-tolerance causes various autoimmune diseases", J Immunol (1995) 155(3): 1151-1164, describes that Treg cells constitutively express the high-affinity IL-2 receptor CD25 at high density, which results in Treg cells positively responding to tiny amounts of IL-2.

DeOca et al., Frontiers in Immunology, Sept 2020, vol. 11, article 541619 describes a fusion protein containing linked CD25 and IL2, which exhibited transient binding to transmembrane CD25 of human embryonic kidney cells (HEK). The fusion protein was suitable for supporting long-term Treg cell cultivation and were found to lack therapeutic efficacy in experimental autoimmune encephalomyelitis.

Dong et al. "The effects of low-dose IL-2 on Treg adoptive cell therapy in patients with Type 1 diabetes", JCI Insight. 2021;6(18):e147474, found that low-dose IL-2 expands exogenously administered Tregs but also can expand cytotoxic cells.

EP 3 478 710 B1, EP 3 865 506 A1 and WO2020/201230 A1 describe CARs that are suitable for use in specifically suppressing HvG disease for transplants having certain HLA molecules.

Noyan et al., Eur J. Immunol. (2014) 44: 2592-2602 "Isolation of human antigen-specific regulatory T cells with high suppressive function" describes that highly pure fractions of alloantigen-specific Treg cells can be selected as CD4+, CD25high, FOXP3+, and additionally be selected as CD154- and LAP+ and/or GARP+, further optionally CD137+ and/or CD127low.

Tenspolde et al., "Regulatory T cells engineered with a novel insulin-specific chimeric antigen receptor as a candidate immunotherapy for type 1 diabetes", Journal of Autoimmunity 103 (2019) 102289, describes that natural Treg cells were isolated by FACS as CD4+, CD25high, and that CD4+ T cells were converted to Treg cells by expression of FOXP3 from a virally transduced Foxp3 encoding plasmid. The transducing plasmid encoded an insulin-specific CAR as a fusion protein with FOXP3, with an intermediate P2A proteolytic site. CD4+ T cells that express FOXP3 from a retrovirally transduced plasmid were identified as regulatory T-cells and hence were termed converted Treg (cTreg) cells.

Gillis and Smith "Long term culture of tumour-specific cytotoxic T cells", Nature (1977), 268(5616), 154-156 describe highly IL-2 dependent CTLL-2 cells.

Spence, A. et al., "Targeting Treg signaling for the treatment of autoimmune diseases", Curr Opin Immunol 37, 11-20 (2015) describes that the CNI Tacrolimus impairs Treg cells in a dose-dependent manner by directly inhibiting Treg activation.

Noyan, F. et al. "Prevention of Allograft Rejection by Use of Regulatory T Cells With an MHC-Specific Chimeric Antigen Receptor", Am J Transplant 17, 917-930 (2017) describes an assay for analysing the suppression activity of CAR-expressing Treg cells.

### Object of the invention

In view of the dependence of Treg cells on IL-2, and the dominance of secretion of IL-2 by T-effector cells which generally cause effects that are antagonistic to the activity of Treg cells, it is an object of the invention to provide for maintenance of Tregs cells that preferably express a CAR in an environment that is low-dose or essentially free from IL-2 and/or in an environment caused by an immune suppressant, without also activating cytotoxic T-cells, e.g. without activating T-effector cells. Preferably, the invention shall provide for maintenance of only Treg cells that were treated for maintenance of Treg cells, which optionally express a CAR, and which are localized by presence of the cognate antigen, for which e.g. their CAR is specific, or for maintenance of Treg cells that are antigen-specific by nature.

### Description of the invention

The invention achieves the object by the features of the claims, and especially by a fusion protein and a nucleic acid construct encoding the fusion protein, which fusion protein comprises or consists of an optional secretory leader peptide, IL-2, preferably a linker peptide, and a membrane-spanning anchor, which fusion protein is also termed membrane-bound IL-2, or mbIL-2. Further, the invention provides an in vitro process for generating Treg cells by genetic manipulation of Treg cells to express the membrane-bound IL-2, and preferably to also express a CAR

Therein, the membrane-bound IL-2 from its N-terminus to its C-terminus comprises or consists of a secretory leader peptide (SP), IL-2, a linker, and a membrane-spanning transmembrane domain and an anchor peptide, preferably having amino acid sequences of at least 90% homology or identity to SEQ ID NO: 1. The secretory leader peptide can be the natural secretory leader peptide of human IL-2, the transmembrane domain and the anchor peptide can e.g. comprise or consist of, from N-terminus to C-terminus, an MHC I bridge, an MHC I transmembrane domain, and an MHC I anchor. Preferably, the linker connecting IL-2 to the transmembrane domain and the anchor peptide has a length of 10 to 20 amino acids, more preferably of at least 12 amino acids or at least 15 amino acids, e.g. in each case up to 18 or up to 16 amino acids. Preferably, the linker contains at least 3 sections, each containing at least 3, more preferably at least 4 consecutive glycines (Gly4), preferably with a C-terminally or N-terminally adjacent Serin (Ser), e.g. the linker contains a Gly₄Ser (Gly4Ser) section and one or two Gly₃Ser sections, preferably directly adjacent to one another.

Generally, the nucleic acid sequence encoding the membrane-bound IL-2 corresponds to a cDNA, especially it is devoid of an intron sequence and/or splice site, both for the fusion protein for membrane-bound IL-2 and for a joint fusion protein comprising both membrane-bound IL-2 and a CAR. A joint fusion protein containing, from N-terminus to C-terminus, a preferred CAR, a P2A site, and membrane-bound IL-2, has the amino acid sequence of SEQ ID NO: 2. The preferred CAR for expression in combination with expression of membrane-bound IL-2 in T-effector cells, preferably in Treg cells, has an amino acid sequence of amino acids 22..512 of SEQ ID NO: 2, preferably with an N-terminal signal sequence, e.g. of amino acids 1..21 of SEQ ID NO: 2. The CAR is connected by a protease 2A site (P2A) to the membrane-bound IL-2 (IL-2). The membrane-bound IL-2 generally preferably from N-terminus to C-terminus has a signal peptide of amino acids 535..554 of SEQ ID NO: 2, an IL-2 of amino acids 555..687 of SEQ ID NO: 2, a linker of amino acids 688..711 of SEQ ID NO: 2, a transmembrane domain of amino acids 712..735 of SEQ ID NO: 2 and an anchor domain of amino acids 736..768 of SEQ ID NO: 2, wherein the linker can comprise a Gly-Ser linker section (GS linker section) of amino acids 688..702 containing one Gly₄Ser section and two Gly₃Ser sections, as preferred, directly adjacent to one another.

The CAR comprising amino acids 1..512 of SEQ ID NO: 2, e.g comprised in a joint fusion protein having SEQ ID NO: 2, has specificity for HLA-A^{∗}02 and its expression in Treg cells suppresses adverse immune reactions against HLA-A^{∗}02, e.g. for use in treatment of HvG disease in HLA-A^{∗}02-negative patients having received a transplant that is HLA-A^{∗}02.

Treg cells in the natural setting for their maintenance are strictly depending on secreted IL-2, which is mainly secreted from T-effector cells (Teff).

It has been found that this strict dependence can be broken by an artificial construct leading to membrane-bound IL-2 expressed by Treg. The localised binding of IL-2 to the cell membrane further bypasses the activation of other IL-2-dependent cells.

In an embodiment, the Treg cells can be antigen-specific Treg cells, which are isolated from a sample, e.g. from an autologous blood sample of a later recipient of the Treg cells. Antigen-specific Treg cells that are already antigen-specific, e.g. due to contact with the antigen, herein are also referred to as Treg cells that are antigen-specific by nature, as they are not genetically manipulated for expression of a CAR. Generally, such Treg cells that are antigen-specific by nature can be defined and isolated, e.g. by FACS, as CD4+ (CD4 positive), CD25high, CD127low, preferably also CD154- (CD154 negative), LAP+ (latency-associated peptide positive) and/or GARP+ (glycoprotein A repetitions predominant positive).

In an embodiment, Treg cells that are not antigen-specific by nature can be isolated as CD4+, CD25high, CD127low, and can optionally be genetically manipulated to express FOXP3 to generate FOXP3+ Treg cells. For antigen-specificity, these Treg cells are genetically manipulated to express an antigen-specific CAR.

Generally preferable, the membrane-bound IL-2 and FOXP3 are encoded in one common nucleic acid construct that is introduced into cells that were isolated to generate Treg cells. For Treg cells that are isolated as CD4+, CD25high, and CD127low, the antigen-specificity of the Treg cells can be generated by genetically manipulating the Treg cells to express a CAR which is specific for the antigen against which the suppressive activity is desired. The CAR and membrane-bound IL-2 can optionally be expressed as separate proteins, e.g. each expressed under the control of a separate promoter, or can be expressed as a joint fusion protein, which contains the CAR and the membrane-bound IL-2, between which a connecting protease site is arranged, wherein the CAR can be arranged N-terminally to the membrane-bound IL-2, or the CAR can be arranged C-terminally to the membrane-bound IL-2. In a joint fusion protein, the CAR and the membrane-bound IL-2 can be arranged in any order, with a connecting protease site between the CAR and the membrane-bound IL-2.

In the alternative to a joint fusion protein containing the CAR and the membrane-bound IL-2, the CAR and the membrane-bound IL-2 can be expressed from one expression cassette under the control of a common promoter with an IRES arranged between the sequences encoding the CAR and the membrane-bound IL-2, and optionally a sequence encoding FOXP3. As a further alternative, each of the CAR and the membrane-bound IL-2 can be expressed from a separate expression cassette, each under the control of its own promoter, which promoter can be the same promoter in each case or a promoter having a different strength.

Generally preferable, the membrane-bound IL-2 and the CAR are encoded on one joint nucleic acid construct that is introduced into Treg cells, which joint nucleic acid construct optionally also encodes FOXP3. In this embodiment, coding sequences for the CAR, for membrane-bound IL-2, and for FOXP3 can be arranged in any order with a coding sequence for a connecting protease site between each, which protease site can be the same in each case or a different one, e.g. different or the same P2A sites. The joint fusion protein can e.g. comprise or consist of CAR-2A-mbIL-2-2A-FOXP3, wherein each protease 2A (P2A) site is the same or a different P2A sequence.

A CAR from its N-terminus to its C-terminus preferably comprises or consists of a single-chain variable fragment antibody domain (scFv), a hinge, a transmembrane domain, and at least one intracellular signalling domain, which preferably are a combination of an intracellular hCD28 signalling domain and an intracellular hCD3zeta domain. Therein, the scFv can be specific for a HLA molecule or SLA molecule for use of the fusion protein in the treatment of HvG disease, or for an autologous antigen for use in the treatment of an autoimmune disease. The autologous antigen is the target antigen against which the autoimmune disease is directed.

The joint fusion protein containing the membrane-bound IL-2 has the advantage of maintaining only those Treg cells that also express the CAR and/or FOXP3, wherein the CAR determines the specificity for the cognate antigen or wherein the Treg cells are antigen-specific by nature, which antigen-specificity, especially which CAR or antigen-specificity by nature, localizes the antigen-specific suppressive activity of the Treg cells to the location in which the cognate antigen is present, e.g. to cells bearing the cognate antigen. Accordingly, the membrane-bound IL-2 in combination with the antigen-specificity of the Treg cells that express the membrane-bound IL-2 results in specific maintenance of genetically manipulated Treg cells, wherein the antigen-specificity of the Treg cells is caused by the expression of a CAR or is caused the natural antigen-specificity, optionally in each case with genetic manipulation of the Treg cells for expression of FOXP3. Accordingly, the antigen-specificity, e.g. due to expression of a CAR as a separate protein or as a joint fusion protein with the membrane-bound IL-2, or due to the isolation of Treg cells that are antigen-specific by nature, results in maintenance of the Treg cells and hence of their antigen-specific suppressive activity, only in the location of the cognate antigen against which suppressive activity is desired.

Further, it has been found that the membrane-bound IL-2 is active to only maintain Treg cells that express the membrane-bound IL-2, e.g. from a nucleic acid sequence encoding membrane-bound IL-2, e.g. a joint fusion protein, and that essentially no T cells, especially no Teff cells, other than those expressing the membrane-bound IL-2 are maintained and can be activated, which is believed to be caused by expression of the membrane-bound IL-2 not resulting in secretion of free IL-2, e.g. no IL-2 being liberated from the Treg cells expressing the membrane-bound IL-2 by hydrolysis or shearing off.

The membrane-bound IL-2 further has the advantage of increasing the proportion and the number of Treg cells expressing the membrane-bound IL-2, optionally in combination with expressing a CAR and/or FOXP3 in relation to conventional Treg cells that are not genetically manipulated. This shows that the expression of the membrane-bound IL-2 only maintains those Treg cells that were genetically manipulated to express the membrane-bound IL-2, which Treg cells were isolated, optionally generated by expression of FOXP3, which Treg cells optionally also express the specific CAR, which provides for the suppressive activity of the Treg cells in the presence of the cognate antigen for which the CAR is specific, but no general enhancement of non-specific Treg cells. The genetic manipulation of Treg cells for expression of membrane-bound IL-2 in IL-2 deprived conditions has the advantage of survival of only Treg cells that express the membrane-bound IL-2, whereas in IL-2 deprived conditions natural Tregs that are not expressing membrane-bound IL-2 are depleted or eliminated. For use of the Treg cells expressing the membrane-bound IL-2 in medical treatment, IL-2 deprived conditions can e.g. be caused by an immune suppressant for use in the treatment in combination with Treg cells expressing the membrane-bound IL-2.

Preferably, the membrane-bound IL-2 is for use in the treatment of HvG disease in the presence of an immunosuppressant, especially a calcineurin inhibitor (CNI).

The invention is now described in greater detail by way of examples with reference to the figures, which show in
- Fig. 1A a schematic representation of an embodiment of a coding sequence of a joint fusion protein including the membrane-bound IL-2 of the invention,
- Fig. 1B FACS results of expression of the membrane-bound IL-2 in HEK293 T-cells,
- Fig. 2A survival of CTLL-2 cells transduced with mbIL-2 CAR or untransduced (WT) CTLL-2 cells, cultured in presence of exogenous IL-2 and under IL-2 limiting conditions,
- Fig. 2B survival advantage of mbIL-2 CAR CTLL-2 cells relative to CTLL-2 WT as a proportion of transduced cells in a competitive proliferation assay under IL-2 free conditions,
- Fig. 3A a schematic representation of the experiment,
- Fig. 3B the change of the proportion of transduced cells expressing comparative control CAR (CTR CAR) or expressing the joint fusion protein of the invention in different concentrations of added IL-2,
- Fig. 3C measured concentrations of IL-2 in medium with added 1000 IU IL-2/ml (1000 IU IL-2/ml) or without added IL-2 (0 IU IL-2/ml) after cultivation of control CAR expressing Treg cells (CTR CAR) or of Treg cells expressing the joint fusion protein (mbIL-2 CAR),
- Fig. 3D FACS results of co-cultivation of Treg cells expressing the control CAR or the joint fusion protein (mbIL-2 CAR) in co-culture with nTreg cells,
- Fig. 4A FACS results for analysis of STAT5 activation by phosphorylation (pSTAT5) for Treg cells expressing the reporter representing the CAR, for nTreg cells and for Treg cells expressing the control CAR (CTR CAR), and for Treg cells expressing the membrane-bound IL-2 of the invention and the CAR,
- Fig. 4B a graph summarizing that only presence of IL-2 in nTregs or in absence of IL-2 the expression of membrane-bound IL-2 results in effective phosphorylation of STAT5 (pSTAT5),
- Fig. 4C a graph of concentrations of release of the immunomodulatory cytokine IL-10 from Treg cells expressing the membrane-bound IL-2 in absence of IL-2 from medium,
- Fig. 5A percentages of FOXP3high Treg cells expressing the control CAR or expressing the membrane-bound IL-2 and the CAR in medium with limiting IL-2 and in IL-2-free medium over 21d culture,
- Fig. 5B the proportion of FOXP3high Treg cells expressing a control CAR only and in Treg cells of the invention at day 21 (d21) of the culture,
- Fig. 5C representative FACS results for FOXP3 and expression of the CAR, represented by the reporter ΔLNGFR, in control Treg cells (CTR CAR Tregs) or in Treg cells expressing the membrane-bound IL-2 (mbIL-2 CAR Tregs),
- Fig. 5D a graph summarizing expression of FOXP3 in Treg cells expressing the control CAR (left columns, CTR CAR) or expressing the joint fusion protein (mbIL-2 CAR) in presence of IL-2-free medium (Medium), in IL-2 free medium containing inflammatory cytokine mix 1 (Mix 1) or in IL-2 free medium containing inflammatory cytokine mix 2 (Mix 2)
- Fig. 6A results of an in vitro proliferation assay in presence of Tacrolimus,
- Fig. 6B a graph summarizing that in mbIL-2 CAR expressing Tregs, the Tacrolimus-induced proliferation inhibition was overcome by expression of the membrane-bound IL-2,
- Fig. 7A a schematic of the experimental set-up,
- Fig. 7B FACS analyses for the indicated time points of the experimental set-up, with boxed regions indicating CAR-expressing Treg cells at competitive re-population (0.15 CTR CAR, 0.15 mbIL-2 CAR) and, following antigen stimulation on Day 1 (D1) at day 11 (D11), showing that only the mbIL-2 CAR expressing Treg cells (0.22) survived (CTR CAR 0.00),
- Fig. 7C a graph summarizing the percentage of CAR expressing spleenic CD4+ cells as represented by the reporter (CAR Treg marker) for control (CTR CAR) and for Treg cells expressing both the membrane-bound IL-2 and the CAR (mbIL-2 CAR),
- Fig. 8A a schematic of the experimental set-up,
- Fig. 8B FACS analyses for the indicated time points of the experimental set-up, with boxed regions indicating CAR-expressing Treg cells [%] at competitive re-population (0.06 CTR CAR, 0.08 mbIL-2 CAR) and, following antigen stimulation on Day 1 (D1), with daily administrations of Tacrolimus, at day 11 (D11), showing that only the mbIL-2 CAR expressing Treg cells (0.45%) could proliferate (CTR CAR 0.07%),
- Fig. 8C a graph summarizing the percentage of CAR expressing spleenic CD4+ cells as represented by the reporter (CAR Treg marker) for control (CTR CAR) and for Treg cells expressing both the membrane-bound IL-2 and the CAR (mbIL-2 CAR) in presence of Tacrolimus.

Generally herein, the cell culture medium was free from IL-2, except for added IL-2. Accordingly, the concentration of IL-2 herein is given as the concentration of IL-2/ml medium as adjusted by initially adding IL-2 to the medium.

### Example 1: Expression of membrane-bound IL-2 as a joint fusion protein with a CAR

As a representative of membrane-bound IL-2, a joint fusion protein of a CAR with the membrane-bound IL-2 with a connecting protease site 2A (P2A) in-between was expressed from a nucleic acid construct that was introduced into cells as a retroviral vector.

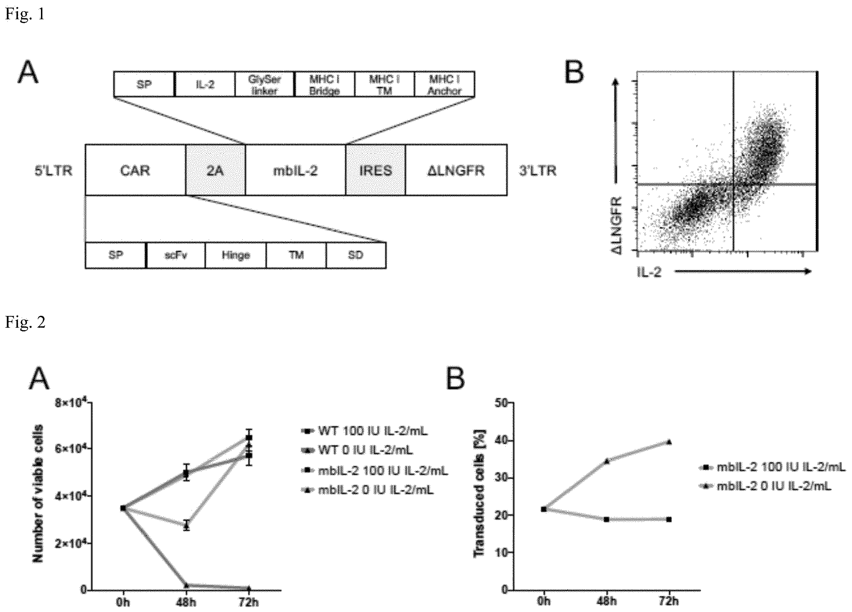

Fig. 1A shows a schematic overview of a nucleic acid construct arranged between a retroviral 5'LTR and a retroviral 3'LTR, the nucleic acid construct encoding from 5' to 3' a joint fusion protein comprising a CAR, a connecting protease site 2A (2A), the membrane-bound IL-2, and an internal ribosome entry site (IRES) controlling expression of a coding sequence for a reporter peptide (ΔLNGFR). The membrane-bound IL-2 consists of a secretory leader peptide (SP), IL-2, a linker (GlySer linker) and a membrane-spanning anchor, which in this embodiment consists of an MHC I bridge (MHC I Bridge), an MHC I transmembrane domain (MHC I TM), and an MHC I anchor domain (MHC I Anchor). The nucleic acid construct preferably has the nucleic acid sequence of SEQ ID NO: 2. The CAR consists of a secretory leader peptide, an scFv, which is exemplified by an scFv having specificity for HLA-A^{∗}02, a hinge (Hinge), a transmembrane domain (TM) and a signalling domain (SD) exemplified by hCD28 signalling domain and the hCD3zeta signalling domain.

As an alternative embodiment, the nucleic acid sequence encoding the CAR and the nucleic acid sequence encoding the membrane-bound IL-2 were each expressed from separate expression cassettes having their own promoter or the expression cassette arranged in 5' having a promoter and the expression cassette arranged in 3' thereto having an IRES for generation of a separate mRNA.

The nucleic acid construct was transduced into HEK293 T-cells by retroviral transduction, alternatively cells were transfected with the nucleic acid construct by use of Lipofectamine.

For γ-retroviral transduction, isolated Tregs were first stimulated with plate-bound α-CD3 (5 µg/mL, UCHT1, BioLegend) and soluble α-CD28 (5 µg/mL, CD28.2, BioLegend) in complete media for 48 hours. Before transduction, protamin sulfate (4µg/ml, Sigma Aldrich) was added to Treg cultures. Tregs were spin-infected at 800 × g at 37 °C for 1 h with retroviral particles. CD4+ CD25high CD127low CD45RA+ nTregs transduced with particles encoding the HLA-A^{∗}02 CAR and membrane bound IL-2 were referred to as mbIL-2 CAR-Tregs and those transduced with particles encoding solely HLA-A^{∗}02 CAR were referred to as CTR CAR-Tregs.

For determination of surface antigens, cells were washed and stained in PBS containing 0.5 % w/v BSA and 2mM EDTA and monoclonal antibodies. For intracellular FOXP3 staining, the Foxp3 Transcription Factor Staining Buffer Set (eBioscience) was used according to the manufacturer's instructions with α-FOXP3 (PCH101, eBioscience). The pSTAT5 stain was performed by directly resuspending cultured cells for fixation in 1.5 % formaldehyde for 10 min at RT followed by permeabilization in ice-cold methanol for 10 min at 4 °C. Cells were stained with α-pSTAT5 (47/Stat5pY694, BD) as described above. Stained cells were analyzed on LSR II (BD) or CytoFLEX (Beckman Coulter) flow cytometers. Flow cytometry FCS files were analyzed using FlowJo Software (version 10, BD).

Fig. 1B shows FACS (fluorescence assisted cell sorting) results using immunological staining for the reporter peptide ΔLNGFR and membrane-bound IL-2 on living HEK293 T-cells that were transduced with the nucleic acid construct of SEQ ID NO: 2. For setting the gates, a comparative nucleic acid construct lacking the membrane-bound IL-2 and the protease site 2A, only encoding the CAR and the IRES controlling the reporter peptide, was transduced into HEK293 T-cells. In the FACS analysis, the gating was set to the right and upper borders of the analysis obtained for the cells containing the comparative construct. The result for the cells transduced with the nucleic acid construct encoding the joint fusion protein of CAR - A2 - membrane-bound IL-2 shows expression of cell surface-bound expression of IL-2 and of the ΔLNGFR reporter. This shows that the membrane-bound IL-2 was expressed as a cell surface-bound IL-2 from a joint fusion protein with a CAR, with concurrent expression of the separate reporter peptide from the same nucleic acid construct.

### Example 2: Expression of membrane-bound IL-2 cultivated in absence of soluble IL-2

For validation of the biological IL-2 activity of the membrane-bound IL-2, the joint fusion protein of Example 1 was retrovirally transduced into CTLL-2 cells, which are highly dependent on IL-2, and 48 h after the transduction the CTLL-2 cells were extensively washed to remove any free IL-2, and then cultured for an additional 72 h in medium without added IL-2 or with 100 IU/mL added IL-2. For comparison, unmodified CTLL-2 cells (wild-type) were cultured under the same conditions. The results are depicted in Fig. 2A, showing that in without added IL-2 to the culture medium (WT 0 IU IL-2/mL), the wild-type CTLL-2 cells died (after 72h, calculated to 9.2 × 10² ± 4.0 × 10²), which also indicates that the original medium was devoid of IL-2. In presence of 100 IU/mL added IL-2, the wild-type CTLL-2 could increase their cell number. The transduced CTLL-2 cells increased their cell number over 72 h both in medium without IL-2 (mbIL-2 0 IU IL-2/mL) and in medium with added IL-2 (mbIL-2 100 IU IL-2/mL) approximately as well as CTLL-2 wild-type cells in medium with added IL-2 (WT 100 IU IL-2/mL), after 72 h calculated to 6.2 × 10² ± 3.5 × 10². This result shows that the membrane-bound IL-2 was expressed and could effectively replace soluble exogenous IL-2. Error bars in Fig. 2A illustrate ± SD.

For direct comparison of the efficacy of membrane-bound IL-2, CTLL-2 cells transduced for expression of membrane-bound IL-2 and wild-type CTLL-2 cells were seeded in a 1:4 ratio (mbIL-2:wild-type) in the same volume of medium, which was either devoid of IL-2 (0 IU IL-2/mL) or contained 100 IU IL-2/mL. Fig. 2B shows that the original ratio (Transduced cells [%]) of mbIL-2 expressing CTLL-2:wild-type CTLL-2 essentially remained constant in the medium containing added IL-2, whereas cultivation in medium that was originally devoid of IL-2 resulted in a drastic shift of the ratio in favour of the mbIL-2 expressing CTLL-2, from an initial ratio of 1:4 to 1:1.5. This result shows that the expression of the membrane-bound IL-2 in CTLL-2 cells, which are highly dependent on IL-2, provided important IL-2 signalling for cell survival.

### Example 3: Maintenance of Treg cells by expression membrane-bound IL-2 in absence of soluble IL-2

For validation of the effect of membrane-bound IL-2 expression in Treg cells, nTreg cells were isolated from a human blood sample by FACS as CD4+, CD25high, CD127low, CD45RA+ cells, expanded in cell culture medium containing IL-2. These nTreg cells were transduced to express the joint fusion protein of Example 1, which are therefore termed mbIL-2 CAR Tregs.

Human specimens were obtained from different HLA-typed healthy donors. Local ethical committee approval was received for the studies. Informed consent of all participating subjects was obtained.

In detail, human natural Tregs (nTregs: CD4+ CD25high CD127low CD45RA+) were isolated from human peripheral blood mononuclear cells (PBMCs) after Ficoll (GE Healthcare) gradient separation and CD25 pre-enrichment (CD25 MicroBeads II, MiltenyiBiotech) via AutoMACS (MiltenyiBiotech). For fluorescence-activated cell scanning (FACS) PBMCs were labelled with monoclonal antibody combinations: CD4+(RPA-T4, BioLegend), CD25+ (2A3, BD), CD127- (hIL-7R-M21, BD), CD45RA+ (MEM-56, ThermoFisher). HLA-A^{∗}02 status was confirmed by flow cytometry via α-HLA-A2/A28 (REA 142, Miltenyi Biotec). Obtained purity of isolated Treg cells was >95%. Tregs were kept in TexMacs GMP cell culture medium (MiltenyiBiotech) supplemented with 10% human AB Serum, 1% Penicillin-Streptomycin (Gibco), ImM sodium pyruvate, 1% non-essential amino acids (NEAA, Gibco) 20mM Hepes and 50µM beta-mercaptoethanol with indicated concentrations of IL-2 (Proleukin, Clinigen). Tregs were expanded by using Treg expansion beads (Miltenyi Biotech) according to manufacturer's instructions.

For the CAR-Treg cells starvation assay, isolated Tregs were transduced with either the mbIL-2 CAR or CTR CAR and. On day 0, cells were washed, seeded and activated with the human Treg Expansion Kit (Miltenyi Biotech) according to the manufacturer's instructions but with different amounts of IL-2: either 1000, 25 or 0 IU IL-2/mL. On days 7, 14 and 21 cells were counted, stained for viability (Fixable Viability Dye, eBioscience), α-CD4 (RPA-T4, BioLegend; SK3, BD), transduction marker (α-CD271/LNGFR, ME20.4, BioLegend; α-Human IgG,F(ab')2, polyclonal, Jackson Immuno Research) and α-FOXP3 followed by flow cytometric analysis.

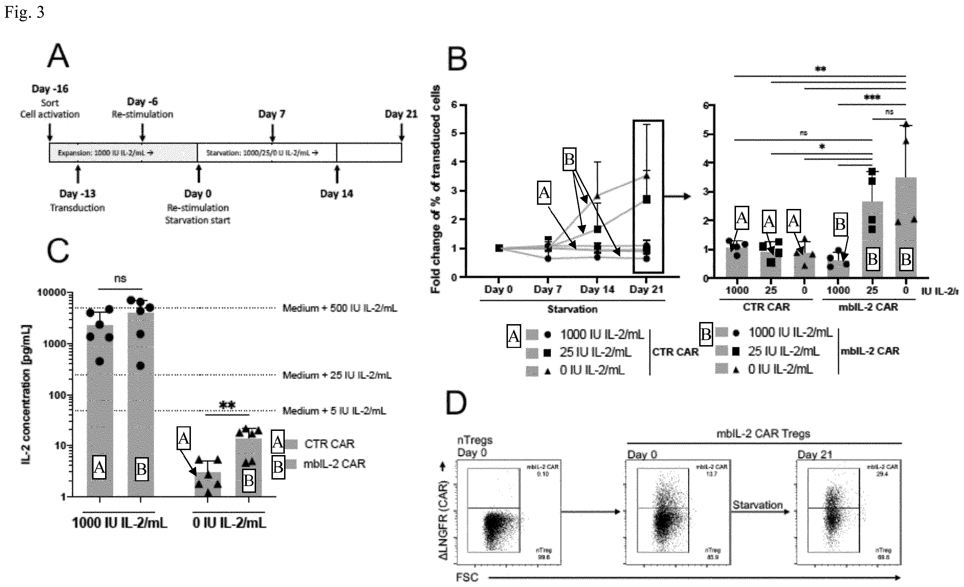

To evaluate the biological effect of mbIL-2 expression in Treg cells, mbIL-2 CAR Tregs were kept in expansion co-cultures with nTregs under various conditions with different IL-2 concentrations. Analysis was performed at various time-points by comparing the cell number and the proportion of mbIL-2 CAR-Tregs. Fig. 3A depicts the experimental set-up of sorting, transducing and expanding mbIL-2 CAR Tregs, and for comparison Treg cells (control, CTR CAR) that were transduced with an expression cassette encoding the CAR (amino acids ... to .. of SEQ ID NO: 1) without the fused membrane-bound IL-2. Cells were counted, stained and analyzed for percentage of transduced cells as well as FOXP3 expression on indicated days.

While mbIL-2 CAR Tregs had no survival disadvantage under conditions of high exogenous IL-2, their proportion increased significantly under limiting IL-2 concentrations (25 IU/ml, 0 IU/ml). As the cells were expanded for 21 days after transduction under high IL-2, these effects were just significant after 7 days under limiting IL-2 conditions.

Fig. 3B Left shows the analysed fold change of [%] of transduced cells for CTR CAR-Tregs and mbIL-2 CAR-Tregs at the indicated timepoints. Fig. 3B Right shows bars representing a summary of all performed experiments at starvation day 21. mbIL-2 led to an increased proliferation of mbIL-2 CAR-Tregs in IL-2 deprivated or IL-2-free cultures. No difference in fold change was seen in mbIL-2 CAR-Tregs cultured with 25 U (2.70 +/- 0.99) or 0 U IL-2 (3.54 +/- 1.79) added IL-2 in the medium. In direct comparison, CTR CAR-Treg proliferation under non-physiological conditions with 25 U (0.96 +/- 0.31) or 0 U IL-2 (0.89 +/- 0.39) proliferation capacity of IL-2 added to the medium was significantly lower (n=4 of different individuals). Normal distribution was calculated using the Shapiro-Wilk normality test. Significance was calculated with an ordinary one-way ANOVA followed by a Dunnett's multiple comparison. Error bars show mean ± SD. ns = not significant, ^{∗}P 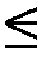 0.05, ^{∗∗}P 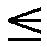 0.005, ^{∗∗∗}P 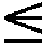 0.001.

Further, the mbIL-2 CAR Tregs were found to remain responsive to high doses of added IL-2 as expansion under 1000 IU/ml of IL-2 was higher than under 25 IU/ml and the mbIL-2 CAR Tregs according to the invention kept their regulative and suppressive capacity.

For analysis of the suppressive activity of CAR-Treg cells, Tregs were isolated, CAR-transduced and expanded. In vitro suppression assays were performed as described by Noyan, F. et al., Am J Transplant 17, 917-930 (2017). CAR-Tregs were labelled with the cell proliferation dye eFluorTM 670 (Thermo Fisher Scientific). 5 ×10⁴ syngeneic CD4+, CD25-effector T cells (Teff) were labelled with carboxyfluorescein diacetate succinimidyl ester (CFSE; 5 mmol/L) and co-cultured with mbIL-2 or CTR CAR-Tregs in various ratios and irradiated 2 ×10⁵ allogeneic HLA-A^{∗}02-positive PBMCs for 5 days. Teff proliferation was calculated based on various Treg:Teff ratios via use of a CFSE dilution assay.

It was analysed whether anchorage of the originally secreted IL-2 by expression of the membrane-bound IL-2 on the cell surface could lead to the spontaneous release of IL-2 from the cell membrane, e.g. by shedding or proteolytic cleavage, which could result in transactivation of Treg cells that do not express the mbIL-2. Analytical results, shown in Fig. 3C, of the supernatant showed as low as 14 pg/ml of soluble IL-2 in the initial IL-2-free cell culture medium from mbIL-2 CAR-Tregs despite 21 days of presence of mbIL-2 CAR Tregs. Measurement of IL-2 concentrations in the supernatant of Tregs cultured in 0 IU IL-2 medium used the BioPlex Cytokine 27-plex Assay system (available from BioRad). Intrapolated medium IL-2 concentrations were calculated using control media with 1000 IU IL-2/mL and 0 IU IL-2/mL. Release of up to 14.33 pg/mL (+/- 7.54) was seen in mbIL-2 CAR-Tregs culture medium. Significance was calculated using an unpaired t-test. Error bars show mean + SD. ns = not significant, ^{∗∗}P 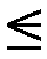 0.01. This concentration is too low to lead to any in vitro expansion of nTregs or of control Treg cells expressing the CTR CAR. This is consistent with the observed growth survival of mbIL-2 CAR Tregs without apparent trans effects on non-transduced nTregs. Under IL-2-free conditions, nTregs rather showed a survival disadvantage despite the presence of co-cultured mbIL-2 CAR Tregs resulting in a decrease of nTreg numbers. This rules out, that mbIL-2 CAR Tregs were capable for an autonomous growth and that shedded IL-2 from their cell surface had a positive effect on nTregs. The FACS results shown in Fig. 3D show the effects of soluble IL-2 concentration in mbIL-2 CAR-Tregs supernatant on nTregs, mbIL-2 CAR-Tregs and nTregs that were cocultured at an original ratio of 1:6 (14% vs. 86%) for 21 days under IL-2-free conditions. FACS analysis at day 21 revealed an increase in mbIL-2 of up to 30% and a concomitant shift in nTreg proportion of up to 70%, corresponding to a decrease in the original ratio to 1:2.3. For Fig. 3D, analysis of the reporter ΔLNGFR represents expression of the CAR from Treg cells expressing the control (CTR CAR), or Treg cells expressing the joint fusion protein mbIL-2 CAR. This shows that with no IL-0 in the medium (Starvation), the percentage of mbIL-2 expressing Treg cells increases from 13.7 to 29.4%, indicating that nTreg cells did not benefit from expression of membrane-bound IL-2 on Treg cells expressing the joint fusion protein.

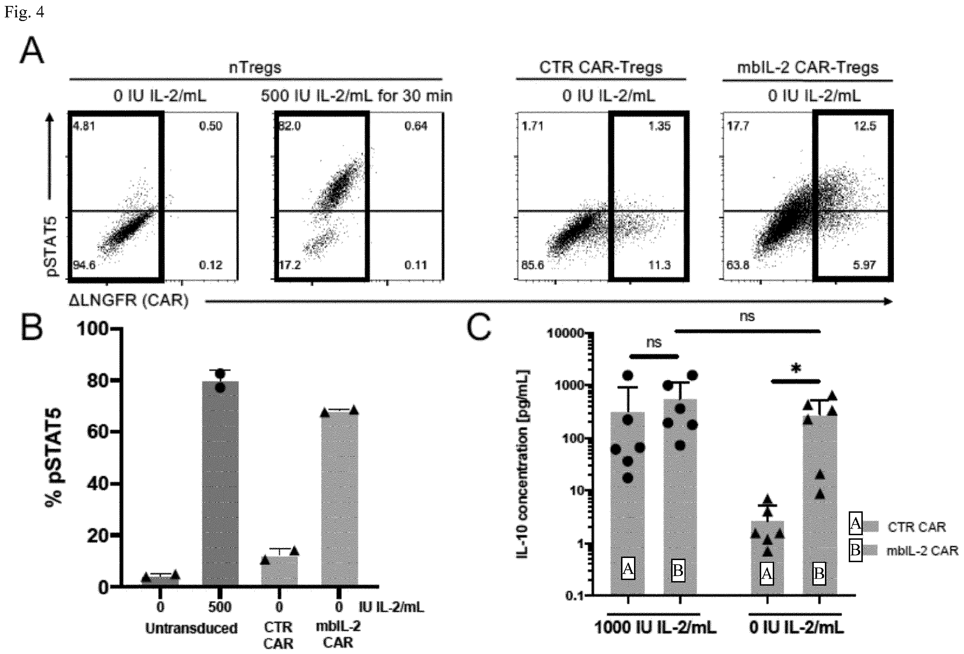

Flow-cytometry (FACS) analyses showed an increase of pSTAT5 under addition of IL-2 to the medium, which was not observed in the absence of IL-2 from the medium. While nTregs transduced with a control CAR (CTR CAR) only did not phosphorylate STAT5, mbIL-2 CAR-Tregs showed a strong STAT5 phosphorylation in the absence of IL-2 from the medium (Fig. 4A, Fig. 4B). Expression of the membrane-associated IL-2 of the invention was thus able to establish the STAT5 signaling pathway in mbIL-2 CAR-Treg cells independently of exogenous IL-2, revealing pathways to stabilize FOXP3 transcription in these Treg cells of the invention. Fig. 4A shows representative dot plots of FACS analyses of an intracellular pSTAT5 staining of nTregs, CTR CAR-Tregs (control) and mbIL-2 CAR-Tregs were cultured under IL-2 free conditions.

Fig. 4B shows that STAT5 activation correlated with IL-2 initiated signalling and could be seen under IL-2 free culture conditions only in mbIL-2 CAR-Tregs (68.18%) and was comparably as high as in nTregs (79.92%) cultured with 500 U IL-2. Absence of IL-2 signaling did not cause STAT5 activation as seen in nTreg with 0 U IL-2 and as well as in CTR CAR-Tregs. The percentage of pSTAT5 was calculated for either untransduced or transduced cells as indicated by emphasized rectangles. n=2.

Fig. 4C shows a bar diagram that summarizes measured concentrations of IL-10 in the supernatant of CTR CAR-Tregs and mbIL-2 CAR-Tregs cultivated under 1000 IU or 0 IU IL-2 added to the culture medium. The absence of exogenous, i.e. added IL-2 conditions (0 IU IL-2 added) resulted in a decrease in IL-10 concentration. The presence of membrane-bound IL-2 in mbIL-2 CAR-Tregs stabilizes the IL-10 concentration even in the absence of IL-2 in the supernatant and is not significantly different from IL-10 concentrations measured in mbIL-2 CAR-Tregs supernatant, cultured with 1000 IU IL-2. The IL-10 concentration was measured using the BioPlex Cytokine 27-plex Assay system (available from BioRad).

Significance was calculated using an unpaired t-test. Error bars show mean + SD. ns = not significant, ^{∗∗}P 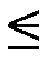 0.01. This shows that expression of membrane-bound IL-2 in Treg cells supports the suppressive mechanism of Tregs of releasing the immunomodulatory cytokine IL-10, whose production is increased by STAT5. Under IL-2-free conditions, membrane-bound IL-2 resulted in increased IL-10 production comparable to that in mbIL-2 cells treated with 500 IU/ml IL-2 (Fig. 4C). In contrast, relevant secretion of this anti-inflammatory cytokine in CTR CAR-Tregs could not be detected.

This shows that the expression of the membrane-bound IL-2 in Treg cells according to the invention results in IL-2 signalling and STAT5 signalling as well als IL-10 signalling comparable to untransduced nTreg cells and to Treg cells expressing the control CAR (CTR CAR) but no membrane-bound IL-2.

It was found that expression of membrane-bound IL-2 resulted in high expression of FOXP3 in mbIL-2 CAR-Tregs and protected them from conversion in the presence of inflammatory cytokines mbIL-2 CAR-Tregs, and further protects against adverse effects of Tacrolimus in vitro.

The levels of FOXP3 expression were analysed in either CTR CAR Tregs and in nTregs or mbIL-2 CAR and nTregs in co-culture assays. This mixed population was divided for the starvation experiment and further cultured under different IL-2 concentrations. On indicated days cells were removed and analyzed by flow cytometry.

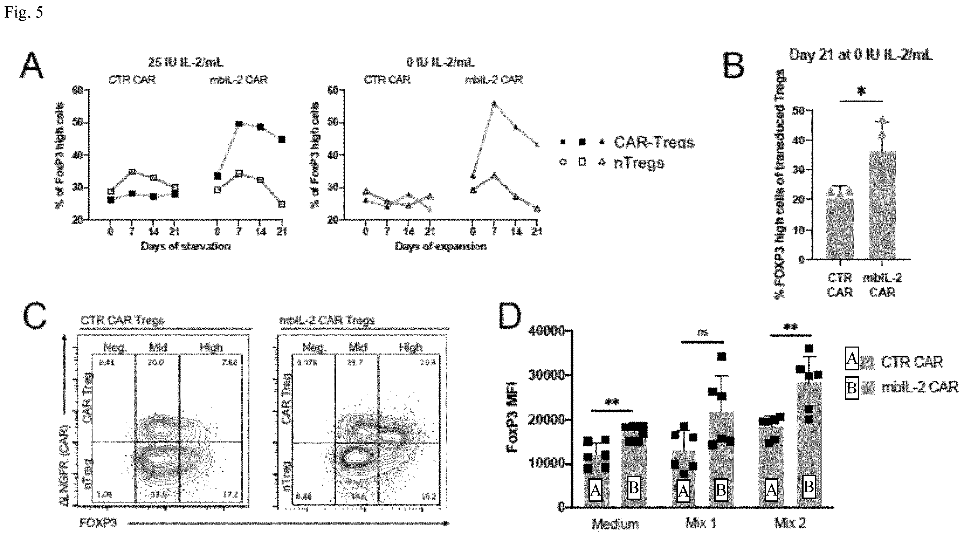

Analyses showed that expression of membrane-bound IL-2 stabilized a high level of FOXP3 expression in Tregs expressing a CAR. The effect of signaling by membrane-bound IL-2 was assessed in relation to FOXP3 expression and FOXP3 stability. The levels of FOXP3 in either mbIL-2 CAR Tregs according to the invention, nTregs, or in CTR CAR expressing Tregs were analyzed in co-culture assays under limiting IL-2 concentrations in the culture medium. The proportion of FOXP3high cells in the nTregs cultured with 25 IU/ml IL-2 initially increased slightly until day 7 but then returned to baseline. Under these conditions, the CTR CAR-Tregs population exhibited no change under 25 IU/ml IL-2 with respect to a FOXP3high population. Likewise, just a minority of FOXP3high cells was observed in CTR CAR-Tregs and nTregs in the absence of IL-2 in the culture medium. In contrast, the mbIL-2 CAR-Tregs of the invention showed a high proportion of FOXP3high cells from day 7 on, both with (25 IU IL-2/ml) and without (0 IU IL-2/ml) Il-2 added to the medium. By day 21, the proportion of FOXP3high cells in the mbIL-2 CAR-Treg compartment of the FACS results had almost doubled (Fig. 5A, Fig. 5B) compared to CTR CAR Treg.

Fig. 5C shows representative FACS plots illustrating the gating strategy of CTR CAR and mbIL-2 CAR-Tregs (0 IU IL-2) of the starvation experiment for transduced and non-transduced Tregs and their classification into FOXP3-negative, -mid and -high cells (Fig. 5C, Neg., Mid, High), with detection of the reporter ΔLNGFR representing expression of the CAR. The levels of FOXP3 expression were analysed in either CTR CAR and nTregs or mbIL-2 CAR and nTregs in co-culture assays. This mixed population was divided for the starvation experiment and further cultured without added IL-2 (0 IU IL-2). The representative FACS plots illustrate the gating strategy of CTR CAR and mbIL-2 CAR-Tregs (0U IL-2) of the starvation experiment for transduced and non-transduced Tregs and their classification into FoxP3-negative, -mid and -high cells. This shows that expression of the membrane-bound IL-2 increased the proportion of both FOXP3+ Treg cells and expression of the CAR in these Treg cells.

In the presence of inflammatory cytokines expression of mbIL-2 protected Tregs from conversion to Teffector cells. FOXP3 expression was investigated after exposure to inflammatory cytokines.

Tregs were isolated and transduced with mbIL-2 CAR or CTR CAR, followed by a resting period of 48 hours at 50 U IL-2/mL. Cells were stimulated with human Treg Expansion Kit (Miltenyi Biotec) and cultivated in Treg medium supplemented with following three different cytokine mixes10: control (10 U/mL IL-2 (Proleukin, Clinigen Inc.), Mix 1 (10 U/mL IL-2 (Proleukin, Clinigen Inc.), 10 ng/mL IL1β (BioLegend), 4 ng/mL IL-6 (BioLegend), 5 ng/mL TGF-β (BioLegend)), Mix 2 (10 U/mL IL-2, 25 ng/mL IL-21 (BioLegend), 25 ng/mL IL-23 (BioLegend), 5 ng/mL TGF-β). After 120 hours cells were harvested, and stained for FOXP3 Fig. 5D shows a bar diagram that summarizes the mean fluorescence intensity (MFI) of FOXP3 expression in CTR CAR and mbIL-2 CAR-Tregs in presence of cytokine mix 1 (IL-2, IL-1β, IL-6, TGF- β) (Mix 1) and cytokine mix 2 (IL-2, IL-21, IL-23, TGF- β) (Mix 2). mbIL-2 CAR-Tregs showed a tendency of higher FOXP3 MFI compared to the CTR CAR-Tregs (2.18 × 10⁴ +/- 8.06 × 10³ vs. 1.30 × 10⁴+/- 4.44 ×10³, p-Value=0.0602). In combination with cytokine mix 2 a significantly higher MFI level was observed in mbIL-2 CAR-Tregs compared to CTR CAR-Tregs (2.83 ×10⁴+/- 5.95 ×10³ vs. 1.83 ×10⁴ +/- 2.54 ×10³). Significance was calculated with one-way ANOVA followed by a multiple comparison. Error bars show mean ± SD. ns = not significant (p=0.0602), ^{∗∗}P 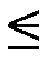 0.005. This shows that Treg cells expressing the membrane-bound IL-2 according to the invention were stable also under cytokine conditions that were comparable to inflammatory conditions.

Further, it was found that mbIL-2 CAR-Treg cells are overcoming the negative effects of Tacrolimus in vitro. CNI, e.g. Tacrolimus (Tac), are part of the standard general immunosuppressive treatment after solid organ transplantation. Tac is known to impair Treg cells in a dose-dependent manner by directly inhibiting Treg activation.

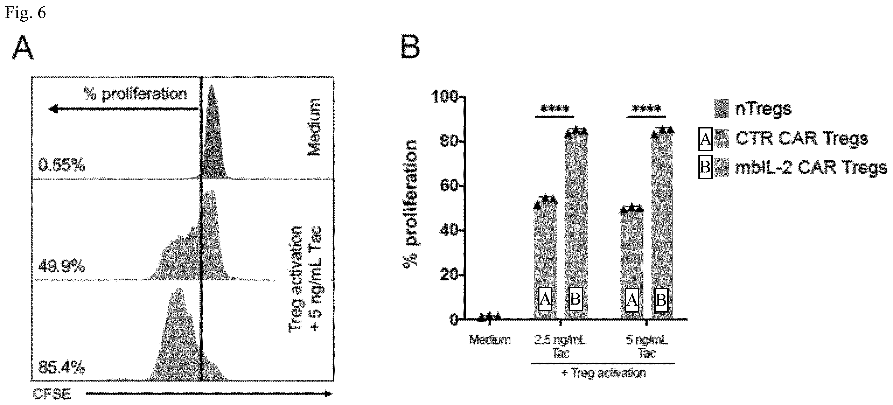

To evaluate the effects of CNIs on Treg proliferation, CFSE-labeled mbIL-2 CAR- and CTR CAR-Tregs were stimulated in the presence of Tacrolimus (Tac) for five days with additional polyclonal stimuli. On day 5, the CFSE dilution was used to determine the cell proliferation (Fig. 6A). CTR CAR-Treg proliferation was reduced in the presence of Tacrolimus. In contrast, CD3/CD28-stimulated mbIL-2 CAR-Tregs showed a much stronger proliferation even in the presence of Tac levels that correspond to clinically relevant levels as seen in patients after transplantation. Treg were isolated and transduced with either the mbIL-2, the CTR CAR or left untransduced followed by expansion as described above followed by cultivation for 48 hours in 20 U IL-2/mL medium for 48 hours. Subsequently cells were stained with CFSE (Thermo Fisher Scientific). nTregs were left in Treg medium as a control, while transduced Tregs were stimulated with the human Treg Expansion Kit (Miltenyi Biotec) according to the manufacturer's instructions plus either 2.5 or 5 ng/mL Tacrolimus (Prograf, Astellas Pharma). After 192 hours, cells were harvested and cell proliferation was measured by flow cytometry.

Proliferative capacity of mbIL-2 CAR-Tregs was significantly higher in the presence of 2.5 and 5 ng/ml Tac than for CTR CAR expressing Tregs. In direct comparison to mbIL-2 CAR-Treg proliferation (85% of proliferating cells) Tac hindered CTR CAR-Treg cell proliferation by more than 40%. Cell proliferation was analyzed and compared to a negative control incubated with only medium (n=3 triplicates, significance was calculated using an unpaired t-test. Error bars show mean + SD. p^{∗∗∗∗} <0.0001). It is remarkable that over 80% of mbIL-2 CAR-Tregs proliferated in the presence of 5 ng/ml of Tac. The bar diagram of Fig. 6B summarizes all performed experiments. Thus, in mbIL-2 CAR-Tregs, the Tacrolimus-induced prevention of NFAT translocation and associated proliferation inhibition was overcome by expression of the membrane-bound IL-2.

This shows that the expression of membrane-bound IL-2 resulted in high expression of FOXP3 in mbIL-2 CAR-Tregs and protected them from conversion in the presence of inflammatory cytokines and protects against adverse effects of Tac in vitro.

### Example 4: Expression of membrane-bound IL-2 enhanced survival of CAR-expressing Treg cells under IIL-2 limiting conditions in vivo and in presence of a CNI

Nonobese diabetic (NOD)-RAG1^{null}IL2c^{null}(NRG) mice aged 8 to 17 weeks were used. All mice were bred and kept under specific pathogen-free conditions. All animal experiments were approved by the local Ethics Animal Review Board and performed in accordance with current German regulations. For in vivo characterization, mbIL-2 CAR-Treg cells were introduced into humanized mice. On day -10, NRG mice were reconstituted with 5×10⁶ HLA-A^{∗}02- PBMCs. Reconstitution was monitored 10 days later post reconstitution by staining for α-CD4 and α-CD8 (SKI, BioLegend) followed by flow cytometric analysis. On day 0, 3 ×10⁵ syngeneic CTR CAR Tregs and mbIL-2 CAR Tregs were injected at a ratio of 1:1. On day 1, irradiated (60Gy) 5 ×10⁶ HLA-A^{∗}02-positive PBMCs irradiated at 60 Gy were transferred. The effect of CNI, exemplified by Tacrolimus, on mbIL-2 CAR-Tregs was assessed in reconstituted NRG-Mice.

Additionally, 5 ×10⁶ HLA-A^{∗}02-positive PBMCs irradiated at 60 Gy were transferred on days 1, 3 and 5, accompanied by daily injections of Tacrolimus (5 mg/kg). Spleens were removed on day 11 and cells were stained for human CD4 and CAR markers (α-IL-2, REA689, Miltenyi Biotec; α-CD34/RQR8, Qbend/10, Invitrogen) and analyzed by flow cytometry.

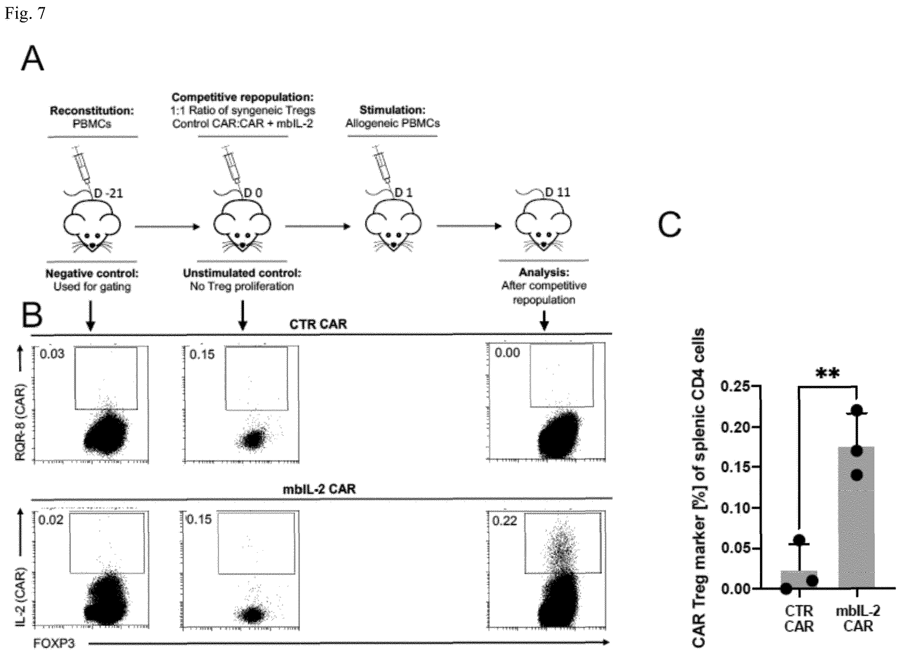

Fig. 7A schematically shows the experimental set-up for analysing the mbIL-2 enhanced survival of CAR-Tregs in an IL-2 limiting environment in this pre-clinical humanized mouse model. On Day -21 NRG mice were reconstituted with HLA-A^{∗}02-negative PBMCs. Differently labeled syngeneic mbIL-2 CAR and CTR CAR-Tregs were adoptively transferred at day 0 in a 1:1 ratio followed by a single injection of allogeneic human HLA-A^{∗}02-positive PBMCs to ensure a CAR specific allogeneic stimulus. Spleens were isolated and analyzed on day 10. Fig. 7B shows representative dot plots measured by FACS, showing the decreasing fraction of CTR CAR-Tregs in the spleens of animals compared with that of mbIL-2 CAR-Tregs in this limiting IL-2 environment. The bar graph of Fig. 7C summarizes the data from all experiments. While a tenfold increase in mbIL-2 CAR-Tregs (0.18% +/-0.04%) could be observed, the number of CTR CAR-Tregs fell below the threshold value (0.02% +/- 0.03%). n=3 of different mice. Significance was calculated using an unpaired t-test. Error bars show mean + SD. p^{∗∗} <0.01.

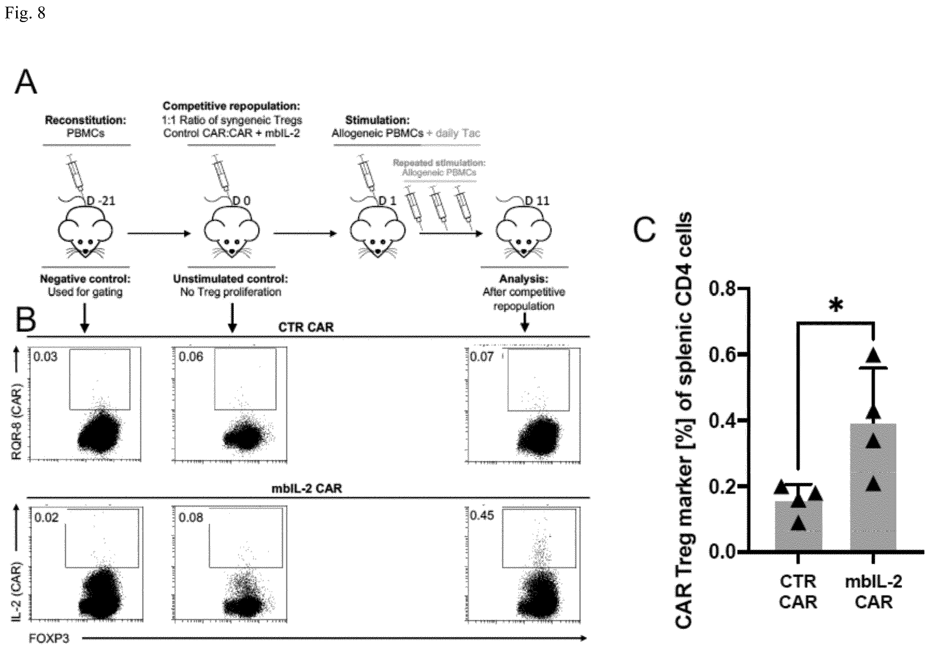

The mbIL-2 enabled CAR-Treg resistance to calcineurin inhibitors was also analysed in a prec-linical humanized mouse model. Fig. 8A shows the experimental setup. On Day -21 NRG mice were reconstituted with HLA-A^{∗}02-negative PBMCs. Differently labeled syngeneic mbIL-2 CAR and CTR CAR-Tregs were adoptively transferred in a 1:1 ratio. To ensure a continued allogeneic stimulus, animals received allogeneic HLA-A^{∗}02-positive PBMCs three times weekly. The scenario of therapy with a CNI for general immune suppression was mimicked by treating the animals daily with Tac (5 mg/kg). This resulted in an IL-2 poor/free environment in vivo. Spleens were isolated and analyzed on day 10.

Fig. 8B gives representative dot plots showing the decreasing fraction of CTR CAR-Tregs in the spleens of animals compared with that of mbIL-2 CAR-Tregs in this example for use of the Treg cells expressing membrane-bound IL-2 in the treatment of HvG disease in the presence of general immune suppression as exemplified by administration of a CNI. In the FACS analyses, the gate for CAR-Tregs was established according to the unstimulated negative control, in animals killed one day after adoptive CAR transfer. It was found that 87% of all adoptively transferred cells could be assigned to the mbIL-2 CAR-Treg cells, which express the membrane-bound IL-2, and 13% to the CTR CAR-Treg cells that express only the CAR. Fig. 8C in the bar graph summarizes the data from all experiments and illustrates the decreasing fraction of CTR CAR-Tregs compared with that of mbIL-2 CAR-Tregs. The proportion of CAR-Tregs that could be assigned to the mbIL-2 modification (0.40% +/- 0.16%) was more than twice that of the CTR CAR proportion (0.16% +/-0.05%). n=4 of different experiments. Significance was calculated using an unpaired t-test. Error bars show mean + SD. p^{∗} <0.05.

### Example 5: Expression of membrane-bound IL-2 as a joint fusion protein with a CAR

As a further example, the CAR encoding nucleic acid sequence and the nucleic acid sequence encoding the membrane-bound IL-2 were expressed from separate expression cassettes. This was the same promoter as used for expression of the fusion protein of Example 1.

The separate expression cassettes encoding the CAR or the membrane-bound IL-2 were integrated into a retroviral vector and packaged in viral particles as described in Example 1.

It was found that the expression of the CAR and of the membrane-bound IL-2, each from separate expression cassettes, in the transduced target cells gave essentially the same results as found for the fusion protein.

### Example 6: Expression of membrane-bound IL-2 in Treg cells that are antigen-specific by nature

Antigenspecific Treg cells were isolated by FACS after allogeneic or xenogeneic stimulus, as CD4+ (CD4 positive), CD25high, CD127low, CD154- (CD154 negative), LAP+ (latency-associated peptide positive) or GARP+ (glycoprotein A repetitions predominant positive). Surface expression of LAP or GARP defines the antigen-specificity.

Alternatively, the antigen-specificity was pre-determined by isolating Treg cells that were not antigen-specific by nature, e.g. as CD4+ (CD4 positive), CD25high, CD127low, CD154+ (CD154 positive), LAP- (latency-associated peptide negative) and GARP-, and incubating these with the antigen against which specificity is desired. Therein, the incubation with the antigen provides an allogeneic or xenogeneic stimulus to the Treg cells, inducing the antigen-specificity in the Treg cells.

As described herein, especially in Example 1, these Treg cells were γ-retrovirally transduced with a nucleic acid construct containing an expression cassette encoding the membrane-bound IL-2 only. As an alternative, the γ-retroviral particle in addition to containing an expression cassette encoding the membrane-bound IL-2 also encoded FOXP3 as a fusion protein, connected by a protease site, or from a separate expression cassette.

It was found that in Treg cells that are antigen-specific by nature, genetic manipulation for expression of membrane-bound IL-2 resulted in antigen-specific Treg cells that express membrane-bound IL-2 and were viable also in an environment deprived of IL-2, e.g. in an environment deprived of IL-2, with the Treg cells expressing the membrane-bound IL-2 in combination with an immune suppressant for use in the treatment of in the treatment of HvG disease, of GvH disease, or of an autoimmune disease.

## Claims

1. Fusion protein for maintenance of regulatory T-cells (Treg cells) in the absence of exogenous IL-2, **characterized by** comprising, from N-terminus to C-terminus, IL-2, a linker and a membrane-anchor, as membrane-bound IL-2.

2. Fusion protein according to claim 1, **characterized by** the membrane-bound IL-2 comprising a secretory leader peptide linked to the N-terminus of IL-2.

3. Fusion protein according to one of the preceding claims, **characterized in that** the membrane-anchor from N-terminus to C-terminus comprises an MHC I bridge, an MHC I transmembrane domain, and an MHC I anchor domain.

4. Regulatory T-cell comprising a fusion protein according to one of the preceding claims.

5. Regulatory T-cell according to claim 4, **characterized by** comprising a nucleic acid construct encoding the fusion protein.

6. Regulatory T-cell according to one of claims 4 to 5, **characterized in that** the regulatory T-cell is CD4+, CD25high, CD127low, and genetically manipulating the cells to express FOXP3.

7. Regulatory T-cell according to one of claims 4 to 5, **characterized in that** the regulatory T-cell is CD4+, CD25high, CD127low, CD154-, LAP+ and/or GARP+, and has been contacted with an antigen against which suppressive activity is desired.

8. Regulatory T-cell according to one of claims 4 to 7, **characterized by** comprising a nucleic acid construct encoding a CAR and/or FOXP3.

9. Regulatory T-cell according to one of claims 4 to 8, **characterized by** comprising a nucleic acid construct encoding the fusion protein in a joint fusion protein which in addition to the membrane-bound IL-2 comprises a CAR and/or FOXP3, wherein a protease site is arranged between the membrane-bound IL-2 and the CAR and/or the FOXP3.

10. Regulatory T-cell according to one of claims 4 to 9, for use in the treatment of HvG disease, of GvH disease, or of an autoimmune disease.

11. Regulatory T-cell according to one of claims 4 to 10 for use in the treatment of HvG disease, of GvH disease, or of an autoimmune disease, in combination with an immunosuppressant for use in the treatment.

12. Nucleic acid construct for expressing a fusion protein for maintenance of regulatory T-cells in regulatory T-cells, the nucleic acid construct encoding from N-terminus to C-terminus, IL-2, a linker and a membrane-anchor, and optionally a secretory leader peptide linked to the N-terminus of IL-2, as membrane-bound IL-2.

13. Nucleic acid construct according to claim 12, **characterized in that** in addition to the fusion protein the nucleic acid construct encodes a CAR and/or FOXP3.

14. Nucleic acid construct according to claim 13, **characterized in that** the nucleic acid construct encodes the fusion protein as a joint fusion protein which in addition comprises a CAR and/or FOXP3.

15. Nucleic acid construct according to one of claims 12 to 14, **characterized in that** that it is contained in a retroviral particle for use in the treatment of HvG disease, of GvH disease, or of an autoimmune disease.
